(19) **Europäisches Patentamt / European Patent Office / Office européen des brevets**

(11) **EP 4 781 949 A1**

(12) **EUROPEAN PATENT APPLICATION**
published in accordance with Art. 153(4) EPC

(43) Date of publication:
**29.07.2026 Bulletin 2026/31**

(51) International Patent Classification (IPC):
**A61F 2/24** (2006.01)

(21) Application number: **24881547.4**

(86) International application number:
**PCT/CN2024/126038**

(22) Date of filing: **21.10.2024**

(87) International publication number:
**WO 2025/087182 (01.05.2025 Gazette 2025/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.10.2023 CN 202311394285**

(71) Applicant: **Suzhou Hearthill Medical Technology Co., Ltd.**
**Suzhou, Jiangsu 215000 (CN)**

(72) Inventors:
• **SUN, Wei**
  **Suzhou, Jiangsu 215000 (CN)**
• **ZHANG, Xiaoyan**
  **Suzhou, Jiangsu 215000 (CN)**
• **YAN, Xiaoshen**
  **Suzhou, Jiangsu 215000 (CN)**

(74) Representative: **Dai, Simin**
**Reyda IP**
**A073**
**157, Quai du Président Roosevelt**
**92130 Issy-les-Moulineaux (FR)**

(54) **POLYMERIC MITRAL HEART VALVE**

(57) The present invention provides a polymeric mitral heart valve. The polymeric mitral heart valve includes leaflets, a stent frame, and an annulus; the stent frame has a hollow cylindrical structure, and the stent frame includes a first end and a second end; the first end is arranged with three circumferentially spaced strut peaks, with strut valleys formed correspondingly; the annulus is arranged on the outer periphery of the second end of the stent frame, and the annulus forms two protruding sections axially toward the first end at positions circumferentially corresponding to two of the strut peaks and a planar section at the remaining one strut peak. The polymeric mitral heart valve provided by the present invention adopts optimized structures of the annulus and the stent frame, thereby improving the adaptability between the annulus and the native annulus, and enhancing the radial supporting force of the stent frame.

FIG. 2

EP 4 781 949 A1

## Description

### CROSS-REFERENCE TO RELATED APPLICATIONS

The present application claims priority to Chinese Patent Application No. 202311394285.7, entitled "POLYMERIC MITRAL HEART VALVE", filed on October 25, 2023, which is incorporated herein by reference in its entirety.

### TECHNICAL FIELD

The present invention relates to the field of medical devices, and in particular, to a polymeric mitral heart valve.

### BACKGROUND OF THE INVENTION

Mitral regurgitation is generally caused by leaflet prolapse due to degenerative changes in the structure of the mitral valve, such as chordae elongation, chordae rupture, annular dilatation, and leaflet thickening; or it is caused by structural and functional changes of the left ventricle due to ischemic or nonischemic causes. The prevalence of mitral regurgitation increases with age, with approximately 10% of the population over 75 years old suffering from this disease in Western countries. Most patients with moderate or severe mitral stenosis and mitral insufficiency require surgical treatment.

At present, commonly used surgical mitral heart valves in clinical practice are polymeric valves. Polymeric valves exhibit excellent fatigue resistance and superior blood compatibility. Polymeric valves can be fabricated by a variety of forming processes, such as injection molding, dip-coating molding, compression molding, electrospinning, and additive manufacturing. Injection-molded valves have the characteristics of high reproducibility and mass production, and are suitable for industrial production.

Mitral valves generally include a stent frame, leaflets, and an annulus. Due to the complexity of the anatomical structure of the mitral valve, the annulus structure of conventional mitral valves cannot adapt to the native annulus, resulting in excessive protrusion into the left ventricle and obstruction of the left ventricular outflow tract. In addition, the stent frame needs a certain radial supporting force to support the leaflets, while the leaflets must have a certain flexibility to achieve opening and closing. To enhance the radial supporting force of the stent frame, the leaflets and the stent frame are made of materials with different hardnesses and bonded together by high-temperature injection molding. This method requires two-shot injection molding, which not only increases the workload of valve manufacturing but also increases the risk of poor bonding between the stent frame and the leaflets.

Therefore, it is necessary to design a polymeric mitral heart valve to solve the above problems.

## SUMMARY

An embodiment of the present invention provides a polymeric mitral heart valve that adopts optimized structures of the annulus and the stent frame, thereby improving the adaptability between the annulus and the native annulus, enhancing the radial supporting force of the stent frame, and optimizing the performance of the valve.

The polymeric mitral heart valve provided in an embodiment of the present invention comprises leaflets, a stent frame, and an annulus; the stent frame has a hollow cylindrical structure, and the stent frame comprises a first end and a second end; the first end is arranged with three circumferentially spaced strut peaks, with strut valleys formed correspondingly; the leaflets are arranged at the center of the first end, the number of the leaflets is identical to the number of the strut peaks, and the three leaflets are circumferentially arranged to sequentially connect two adjacent strut peaks; wherein the annulus is arranged on the outer periphery of the second end of the stent frame, and the annulus forms two protruding sections axially toward the first end at positions circumferentially corresponding to two of the strut peaks and a planar section at the remaining one strut peak; the valve further comprises an insert having a hollow cylindrical structure matching the stent frame, and the insert is adapted to be axially inserted into the stent frame; the leaflets, the stent frame, and the annulus are formed by one-shot injection molding, and the insert is embedded in the stent frame during injection molding to be formed integrally with the stent frame.

Optionally, the protruding sections are smoothly connected to the planar section, the two protruding sections are smoothly connected to each other, and the minimum height between the two protruding sections is the same as the height of the planar section; the maximum height of the protruding sections is 6 mm to 10 mm; and the height of the planar section is 2 mm to 6 mm.

Optionally, a plurality of axial through holes are circumferentially spaced at an end of the annulus; a sealing membrane closing the end is arranged at the end of the annulus adjacent to the first end; and the sealing membrane is connected to the stent frame and the annulus by sutures.

Optionally, the annulus is formed with a suture base at an end adjacent to the second end; the suture base is adapted to be sutured to a native annulus; and the axial height of the suture base is 0.5 mm to 1.5 mm.

Optionally, the insert has a frame structure matching the outer contour of the stent frame, and protruding fixing ribs and planar fixing ribs are circumferentially arranged on the insert corresponding to the upper edges of the protruding sections and the planar section, respectively; first suture holes are arranged on both the protruding fixing ribs and the planar fixing ribs, and the first suture holes and the through holes are circumferentially arranged in a one-to-one correspondence.

Optionally, the insert comprises a plurality of positioning peaks, a plurality of positioning teeth, and a plurality of positioning protrusions; the positioning peaks are arranged at an end adjacent to the first end, and the plurality of positioning peaks are circumferentially arranged corresponding to the strut peaks; the positioning teeth are arranged at an end adjacent to the second end, and the circumferential positions of the plurality of positioning teeth correspond one-to-one to the strut peaks and the strut valleys; the positioning protrusions are uniformly arranged on the outer wall of the insert.

Optionally, second suture holes are arranged at the tops of the positioning peaks.

Optionally, each leaflet comprises a free edge and an attachment edge connected to the stent frame; in a natural state, gaps are reserved between the free edges of adjacent leaflets, a concave structure is formed between the midpoint of each free edge and the endpoints of the attachment edge, and the leaflets are semi-open.

Optionally, the line connecting the midpoint of the free edge and the midpoint of the attachment edge forms a belly straight line on each leaflet, and the angle between the belly straight line and the cross-section of the stent frame is 30° to 45°.

Optionally, M is defined as the midpoint of the connecting arc extending circumferentially along the stent frame between two endpoints of the attachment edge, and N is defined as the commissural point of the leaflets in a natural state; the line connecting M and N extends obliquely from the first end to the second end, and the angle between the line connecting M and N and the cross-section of the stent frame is 10° to 30°.

Optionally, the free edge of a single leaflet is located in a coordinate plane, where the X-axis of the coordinate system is defined by the line connecting the midpoint of the free edge and one endpoint of the attachment edge, and the Y-axis is perpendicular to the connecting line and passes through the midpoint of the free edge; the parametric curve Y of the free edge from the midpoint to one endpoint of the attachment edge is represented by the following formula:

$$Y = \begin{cases} H_1 \sin\left(\dfrac{X}{L_1}\pi\right) & 0 \leq X \leq L_1 \\[2mm] H_2 \sin\left(\dfrac{X}{L_1+L_2}3\pi\right) & L_1 < X < L_2 \\[2mm] H_3 \sin\left(\dfrac{X}{L_2+L_3}\pi\right) & L_2 \leq X \leq L_3 \end{cases}$$

wherein $L_1$ and $L_2$ are adjustable parameters and satisfy $L_3/10 \leq L_1 \leq L_3/5$ and $2L_3/3 \leq L_2 \leq 3L_3/4$, respectively; $L_3$ is a linear distance between the midpoint of the free edge and one endpoint of the attachment edge; $H_1$, $H_2$, and $H_3$ are adjustable parameters and satisfy $0.05$ mm $\leq H_1 \leq$ 0.20 mm, 0.20 mm $\leq H_2 \leq$ 1.0 mm, and 0 mm $\leq H_3 \leq$ 0.25 mm, respectively.

Optionally, the thickness of the leaflets is 0.2 mm to 0.5 mm.

Optionally, the leaflets, the stent frame, and the annulus are all made of styrene-ethylene-butylene-styrene block copolymer, polyether polyurethane, polyester polyurethane, or silane-modified polyurethane.

Compared with the prior art, the technical solutions in the embodiments of the present invention have beneficial effects.

For example, the annulus of the present invention is provided with protruding sections corresponding to two strut peaks and a planar section corresponding to the remaining one strut peak to adapt to the native mitral valve structure. Compared with an annulus having a uniform height, the arrangement of the protruding sections reduces protrusion of the valve into the left ventricle after implantation, diminishes obstruction to the left ventricular outflow tract, and improves the hydrodynamic performance of the valve.

For another example, an insert is arranged in the stent frame to provide radial supporting force, so that the leaflets, the stent frame, and the annulus can be integrally injection-molded from the same polymer material, thereby optimizing the performance of the valve, simplifying preparation processes, and improving production efficiency.

For another example, protruding fixing ribs and planar fixing ribs are arranged on the insert, and first suture holes corresponding to the through holes of the annulus are provided. The sealing membrane, the stent frame, and the insert can be sutured together by sutures, so as to increase the connection strength between the annulus and the stent frame and enhance the bonding force between the insert and the stent frame. Second suture holes are arranged at the tops of the positioning peaks of the insert for sutures to pass therethrough, so that the stent frame can be bent toward the central axis of the valve during valve implantation to facilitate placement into the native mitral valve structure.

For another example, the leaflets are designed to be semi-open in a natural state, which increases the flow rate therethrough during opening of the leaflets, lowers the commissural point of the leaflets, reduces obstruction of the left ventricular outflow tract, optimizes the structure of the leaflets, reduces valve stress, and prolongs the service life of the valve.

**BRIEF DESCRIPTION OF THE DRAWINGS**

FIG. 1 is a structural view of a native mitral valve.
FIG. 2 is a schematic structural view of a polymeric mitral heart valve according to an embodiment of the present invention.
FIG. 3 is a front view of the polymeric mitral heart valve according to an embodiment of the present invention.

FIG. 4 is a perspective view of the polymeric mitral heart valve according to an embodiment of the present invention.

FIG. 5 is a top view of the polymeric mitral heart valve according to an embodiment of the present invention.

FIG. 6 is an A-A cross-sectional view of FIG. 4.

FIG. 7 is a schematic structural view of an insert according to an embodiment of the present invention.

FIG. 8 is a schematic structural view of a leaflet according to an embodiment of the present invention.

FIG. 9 shows a curve of the free edge of the leaflet in the positive direction of the X-axis according to an embodiment of the present invention.

FIG. 10 shows the leaflet stress distribution of the polymeric mitral heart valve in a closed state according to an embodiment of the present invention.

FIG. 11 shows the leaflet stress distribution of a conventional mitral heart valve in a closed state.

Reference Numeral Description:

1, stent frame; 11, first end; 12, second end; 13, strut peak; 14, strut valley;

2, annulus; 21, protruding section; 22, planar section; 23, suture base; 24, through hole;

3, leaflet; 31, free edge; 32, attachment edge; 33, belly straight line;

4, sealing membrane;

5, insert; 51, protruding fixing rib; 52, planar fixing rib; 53, first suture hole; 54, positioning peak; 55, positioning protrusion; 56, second suture hole; 57, positioning tooth;

101, left ventricle; 102, left atrium; 103, native annulus; 104, commissure of anterior and posterior leaflets; 105, left ventricular outflow tract.

## DETAILED DESCRIPTION OF THE INVENTION

To make the objectives, features, and beneficial effects of the present invention clearer and more comprehensible, specific embodiments of the present invention are described in detail below with reference to the accompanying drawings. It should be understood that the specific embodiments described below are merely illustrative of the present invention, rather than limiting thereto. In addition, the same or similar reference numerals may be used in the drawings to denote the same or similar elements in different embodiments, and descriptions of the same or similar elements in different embodiments, as well as descriptions of elements, features, and effects in the prior art, may be omitted. It should be noted that the central axis, axial direction, radial direction, and circumferential direction described in the embodiments of the present invention mean the central axis, axial direction, radial direction, and circumferential direction of the stent frame 1, respectively.

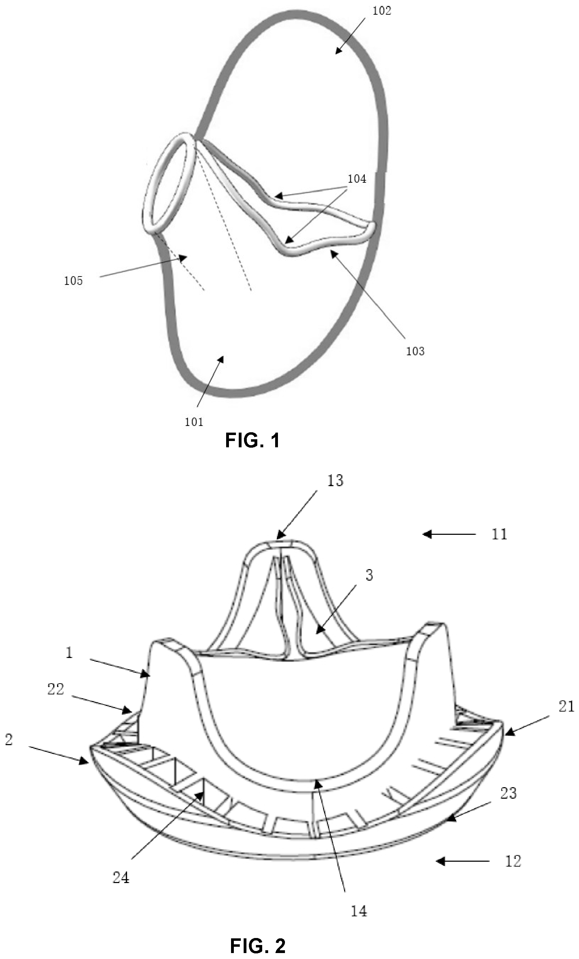

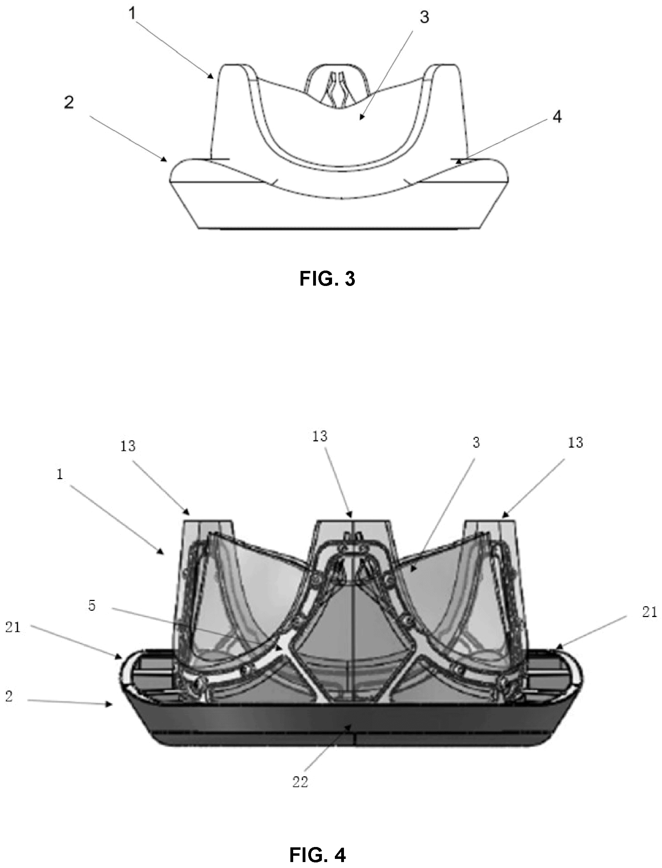

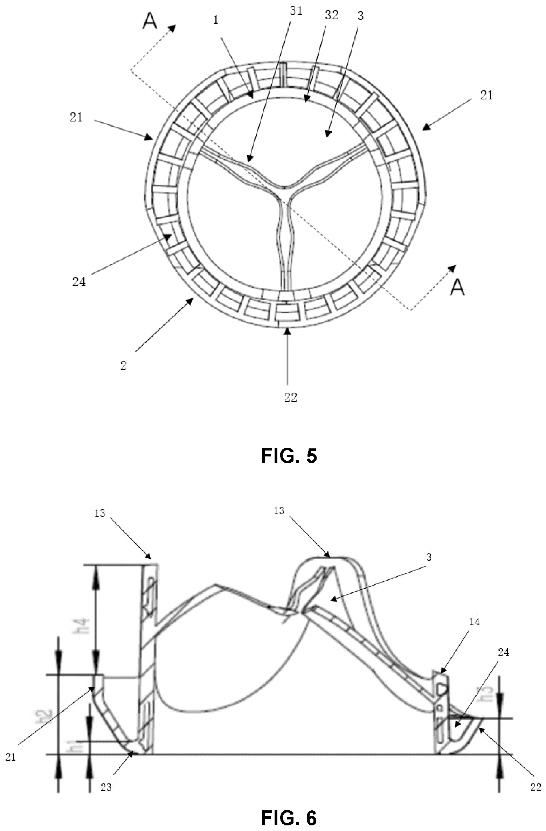

With reference to FIGS. 2 to 10, an embodiment of the present invention provides a polymeric mitral heart valve.

In a specific embodiment, the polymeric mitral heart valve includes leaflets 3, a stent frame 1, and an annulus 2. The stent frame 1 has a hollow cylindrical structure, and the stent frame 1 includes a first end 11 and a second end 12. The first end 11 is arranged with three circumferentially spaced strut peaks 13, with strut valleys 14 formed correspondingly. The leaflets 3 are arranged at the center of the first end 11, the number of the leaflets 3 is identical to the number of the strut peaks 13, and the three leaflets 3 are circumferentially arranged to sequentially connect two adjacent strut peaks 13. The annulus 2 is arranged on the outer periphery of the second end 12 of the stent frame 1, and the annulus 2 forms two protruding sections 21 axially toward the first end 11 at positions circumferentially corresponding to two of the strut peaks 13 and a planar section 22 at the remaining one strut peak 13. The valve further includes an insert 5 having a hollow cylindrical structure matching the stent frame 1, and the insert 5 is adapted to be axially inserted into the stent frame 1. The leaflets 3, the stent frame 1, and the annulus 2 are formed by one-shot injection molding, and the insert 5 is embedded in the stent frame 1 during injection molding to be formed integrally therewith.

FIG. 1 is a structural view of a native mitral valve. With reference to FIG. 1, the native mitral valve is generally saddle-shaped and located between the left ventricle 101 and the left atrium 102. A native annulus 103 is lowest at a commissure 104 of the anterior and posterior leaflets. During replacement of the mitral heart valve, the protruding sections 21 of the polymeric mitral heart valve according to the embodiment of the present invention are correspondingly arranged at the commissure 104 of the anterior and posterior leaflets, so as to reduce protrusion of the stent frame 1 into the left ventricle 101 and diminish obstruction to the left ventricular outflow tract 105.

In a specific embodiment, the protruding sections 21 are smoothly connected to the planar section 22, and the two protruding sections 21 are smoothly connected to each other, so as to be adapted to connect with the native annulus 103. The minimum height between the two protruding sections 21 is the same as the height of the planar section 22. The maximum height h2 of the protruding sections 21 is 6 mm to 10 mm. The height h3 of the planar section 22 is a fixed value, ranging from 2 mm to 6 mm. This enables the annulus 2 of the valve to fit closely to the native annulus 103 as much as possible, while minimizing the protrusion height h4 within the left ventricle 101, thereby reducing outflow tract obstruction.

In some embodiments, a plurality of axial through holes 24 are circumferentially spaced at an end of the annulus 2. The through holes 24 improve the flexibility of the annulus 2, allowing the valve to fit closer to the native mitral valve structure after implantation. Due to the through holes 24, a sealing membrane 4 closing the end is arranged at the end of the annulus 2 adjacent to the first end 11. The sealing membrane 4 is connected to the stent frame 1 and the annulus 2 by sutures.

In some embodiments, the annulus 2 is formed with a suture base 23 at an end adjacent to the second end 12. The suture base 23 is adapted to be sutured to the native annulus 103 by sutures. The axial height h1 of the suture base 23 is 0.5 mm to 1.5 mm. An excessively large axial height h1 is unfavorable for the passage of a suture needle, while an excessively small axial height h1 tends to cause pulling of the annulus 2 when the suture needle passes therethrough, resulting in large gaps at the base.

In some embodiments, the insert 5 has a frame structure matching the outer contour of the stent frame 1. Protruding fixing ribs 51 and planar fixing ribs 52 are circumferentially arranged on the insert 5 corresponding to the upper edges of the protruding sections 21 and the planar section 22, respectively. First suture holes 53 are arranged on both the protruding fixing ribs 51 and the planar fixing ribs 52, and the first suture holes 53 and the through holes 24 are circumferentially arranged in a one-to-one correspondence.

In a specific embodiment, the sealing membrane 4 is connected to the stent frame 1 by sutures passing through the through holes 24 and the first suture holes 53. The sealing membrane 4, the stent frame 1, and the insert 5 are sutured together by sutures, so as to increase the connection strength between the annulus 2 and the stent frame 1 and enhance the bonding force between the insert 5 and the stent frame 1.

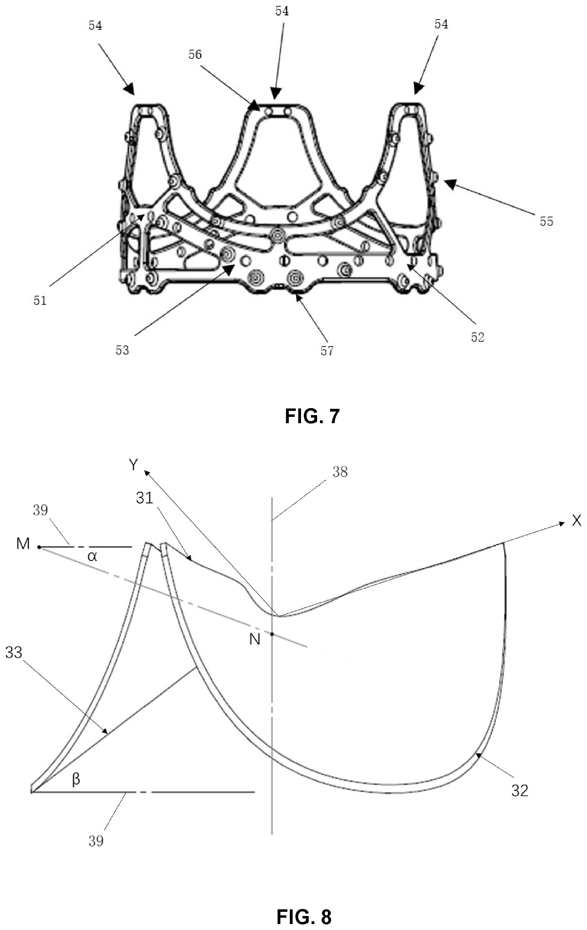

With reference to FIG. 7, in some embodiments, the insert 5 includes a plurality of positioning peaks 54, a plurality of positioning teeth 57, and a plurality of positioning protrusions 55. The positioning peaks 54 are arranged at an end adjacent to the first end 11, and the plurality of positioning peaks 54 are circumferentially arranged corresponding to the strut peaks 13. The positioning teeth 57 are arranged at an end adjacent to the second end 12, and the circumferential positions of the plurality of positioning teeth 57 correspond one-to-one to the strut peaks 13 and the strut valleys 14. Each positioning tooth 57 includes two teeth. The positioning protrusions 55 are uniformly arranged on the outer wall of the insert 5.

In some embodiments, second suture holes 56 are arranged at the tops of the positioning peaks 54. The second suture holes 56 allow sutures to pass therethrough, so that the stent frame 1 can be bent toward the central axis of the valve during valve implantation to facilitate placement into the native mitral valve structure.

In some embodiments, each leaflet 3 includes a free edge 31 and an attachment edge 32 connected to the stent frame 1. In a natural state, gaps are reserved between the free edges 31 of adjacent leaflets 3. A concave structure is formed between the midpoint of each free edge 31 and the endpoints of the attachment edge 32, and the leaflets 3 are semi-open. The semi-open leaflets 3 allow fluid to pass therethrough without applied load, whereas fully closed leaflets 3 in a natural state require a load to deform and open the leaflets to achieve the same flow rate. When a larger load is applied, the semi-open leaflets 3 continuously deform and open on the basis of the semi-open state. Therefore, under the same applied load, the semi-open leaflets provide a higher flow rate than fully closed leaflets, thereby increasing the blood flow passing through the valve during opening. It should be noted that the semi-open leaflets 3 have a commissural point N, which is located on the central axis 38 and between the midpoint of the free edge 31 and the second end 12.

With reference to FIG. 8, in some embodiments, the line connecting the midpoint of the free edge 31 and the midpoint of the attachment edge 32 forms a belly straight line 33 on each leaflet 3, and the angle $\beta$ between the belly straight line 33 and the cross-section 39 of the stent frame 1 is 30° to 45°.

In some embodiments, M is defined as the midpoint of the connecting arc extending circumferentially along the stent frame 1 between two endpoints of the attachment edge 32, and N is defined as the commissural point of the leaflets in a natural state. The line connecting M and N extends obliquely from the first end 11 to the second end 12, and the angle $\alpha$ between the line connecting M and N and the cross-section 39 of the stent frame 1 is 10° to 30°, preferably 20°. When the leaflets 3 are closed, the commissural point N on the leaflets 3 moves toward the second end 12 relative to the open state. That is, in FIG. 1, space is reserved on the right side of the left ventricular outflow tract 105, so as to reduce obstruction of the left ventricular outflow tract 105 when the leaflets 3 are closed. Here, the cross-section 39 is any plane perpendicular to the central axis 38.

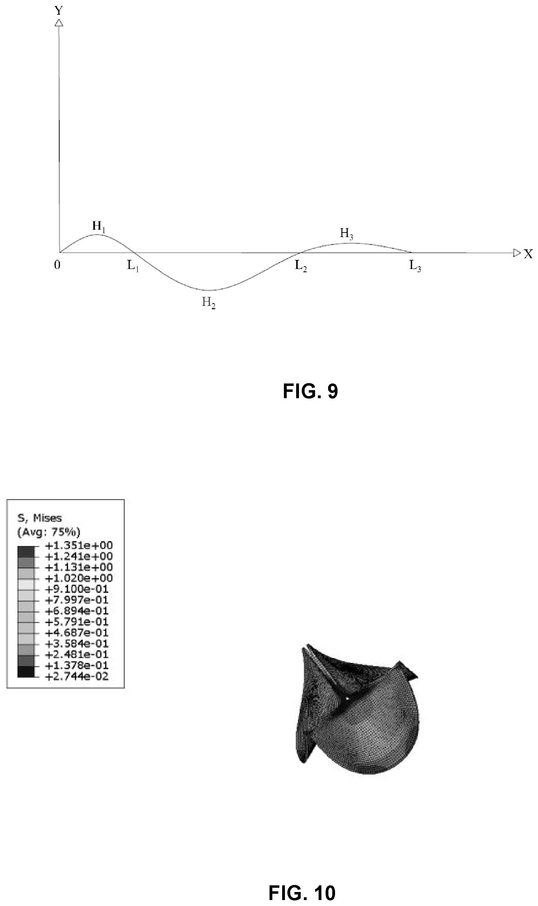

With reference to FIGS. 8 and 9, in some embodiments, the free edge 31 of a single leaflet 3 is located in a coordinate plane, where the X-axis of the coordinate system is defined by the line connecting the midpoint of the free edge 31 and one endpoint of the attachment edge 32, and the Y-axis is perpendicular to the connecting line and passes through the midpoint of the free edge 31. A curve Y of the free edge 31 in the positive direction of the X-axis, i.e., the parametric curve Y of the free edge 31 from the midpoint (i.e., the point where x = 0 and y = 0 in the coordinate system) to one endpoint of the attachment edge 32 (i.e., the point where $x = L_3$ and y = 0 in the coordinate system), is represented by the following formula:

$$Y = \begin{cases} H_1 \sin\left(\dfrac{X}{L_1}\pi\right) & 0 \leq X \leq L_1 \\[2ex] H_2 \sin\left(\dfrac{X}{L_1 + L_2}3\pi\right) & L_1 < X < L_2 \\[2ex] H_3 \sin\left(\dfrac{X}{L_2 + L_3}\pi\right) & L_2 \leq X \leq L_3 \end{cases}$$

wherein $L_1$ and $L_2$ are adjustable parameters and satisfy $L_3/10 \leq L_1 \leq L_3/5$ and $2L_3/3L_2 \leq L_3 \leq 3/4$, respectively; $L_3$ is a linear distance between the midpoint of the free edge 31 and one endpoint of the attachment edge 32; $H_1$, $H_2$, and $H_3$ is adjustable parameters and satisfy $0.05 \text{ mm} \leq H_1 \leq 0.20 \text{ mm}$, $0.20 \text{ mm} \leq H_2 \leq 1.0 \text{ mm}$, and $0 \text{ mm} \leq H_3 \leq 0.25 \text{ mm}$, respectively. The parametric curve Y, in the course of its extension from the midpoint (x = 0, y = 0) of the free edge 31 to one endpoint (x = $L_3$, y = 0) of the attachment edge 32, is sequentially provided with a first peak, a first trough, and a second peak, and $H_1$, $H_2$, and $H_3$ correspond to the y-values at the first peak, the first trough, and the second peak, respectively.

After the curve of the free edge 31 is determined, the belly straight line 33 is obtained according to the angle between the belly straight line 33 and the cross-section of the stent frame 1. The intersection line between the curved surface formed by extending the free edge 31 along the belly straight line 33 and the cylinder formed by the inner diameter of the stent frame 1 is the attachment edge 32. The curved surface enclosed by the foregoing three lines is the shape of leaflet 3.

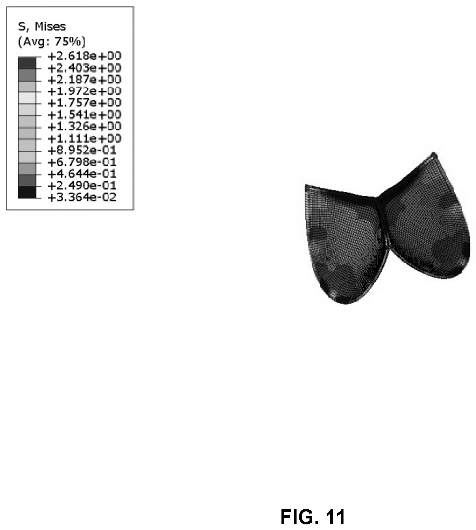

With reference to FIGS. 10 and 11, the leaflet stress distributions are compared under a pressure of 120 mmHg. It can be seen that the maximum stress of the leaflets according to the embodiment of the present invention is 1.351 MPa, while that of conventional mitral valve leaflets is 2.618 MPa, representing a reduction of 93.8% in maximum stress. Such a reduction in maximum stress greatly prolongs the service life of the valve.

In some embodiments, the thickness of the leaflets 3 is 0.2 mm to 0.5 mm. This ensures flexibility and fatigue resistance.

In some embodiments, the leaflets 3, the stent frame 1, and the annulus 2 are all made of a polymer material selected from one or a combination of polyether polyurethane, polyester polyurethane, silane-modified polyurethane, and triblock copolymers.

In summary, the annulus 2 according to the embodiment of the present invention is provided with protruding sections 21 corresponding to two strut peaks 13 and a planar section 22 corresponding to the remaining one strut peak 13 to adapt to the native mitral valve structure. Compared with an annulus having a uniform height, the arrangement of the protruding sections reduces protrusion of the valve into the left ventricle 101 after implantation, diminishes obstruction to the left ventricular outflow tract 105, and improves the hydrodynamic performance of the valve.

Further, in the embodiment of the present invention, an insert 5 is arranged in the stent frame 1 to provide radial supporting force, so that the leaflets 3, the stent frame 1, and the annulus 2 can be integrally injection-molded from the same polymer material, thereby optimizing the performance of the valve, simplifying preparation processes, and improving production efficiency.

Further, in the embodiment of the present invention, protruding fixing ribs 51 and planar fixing ribs 52 are arranged on the insert 5, and first suture holes 53 corresponding to the through holes 24 of the annulus 2 are provided. The sealing membrane 4, the stent frame 1, and the insert 5 can be sutured together by sutures, so as to increase the connection strength between the annulus 2 and the stent frame 1 and enhance the bonding force between the insert 5 and the stent frame 1. Second suture holes 56 are arranged at the tops of the positioning peaks 54 of the insert 5 for sutures to pass therethrough, so that the stent frame 1 can be bent toward the central axis of the valve during valve implantation to facilitate placement into the native mitral valve structure.

Further, the leaflets 3 according to the embodiment of the present invention are designed to be semi-open in a natural state, which reduces obstruction of the left ventricular outflow tract 105, optimizes the structure of the leaflets 3, reduces valve stress, and prolongs the service life of the valve.

Although specific embodiments have been described above, such embodiments are not intended to limit the scope of the present disclosure, even where a single embodiment is described with respect only to specific features. The examples of features provided in the present disclosure are exemplary rather than limiting, unless otherwise stated. In specific embodiments, according to actual requirements and where technically feasible, one or more technical features of the dependent claims may be combined with the technical features of the independent claims. Moreover, the technical features from the corresponding independent claims may be combined in any suitable manner, and are not limited to the specific combinations recited in the claims.

Although the present invention has been disclosed above, the present invention is not limited thereto. Any person skilled in the art may make various changes and modifications without departing from the spirit and scope of the present invention. Therefore, the protection scope of the present invention shall be subject to the scope defined by the claims.

Claims

1. A polymeric mitral heart valve, comprising leaflets, a stent frame, and an annulus; the stent frame has a hollow cylindrical structure, and the stent frame comprises a first end and a second end; the first end is arranged with three circumferentially spaced strut peaks, with strut valleys formed correspondingly; the leaflets are arranged at the center of the first end, the number of the leaflets is identical to the number of the strut peaks, and the three leaflets are circumferentially arranged to sequentially connect two adjacent strut peaks; wherein the annulus is arranged on the outer periphery of the second end of the stent frame, and the annulus forms two protruding sections axially toward the first end at positions circumferentially corresponding to two of the strut peaks and a planar section at the remaining one strut peak; the valve further comprises an insert having a hollow cylindrical structure matching the stent frame, and the insert is adapted to be axially inserted into the stent frame; the leaflets, the stent frame, and the annulus are formed by one-shot injection molding, and the insert is embedded in the stent frame during injection molding to be formed integrally with the stent frame.

2.  The polymeric mitral heart valve according to claim 1, wherein the protruding sections are smoothly connected to the planar section, the two protruding sections are smoothly connected to each other, and the minimum height between the two protruding sections is the same as the height of the planar section; the maximum height of the protruding sections is 6 mm to 10 mm; and the height of the planar section is 2 mm to 6 mm.

3.  The polymeric mitral heart valve according to claim 1, wherein a plurality of axial through holes are circumferentially spaced at an end of the annulus; a sealing membrane closing the end is arranged at the end of the annulus adjacent to the first end; and the sealing membrane is connected to the stent frame and the annulus by sutures.

4.  The polymeric mitral heart valve according to claim 3, wherein the annulus is formed with a suture base at an end adjacent to the second end; the suture base is adapted to be sutured to a native annulus; and the axial height of the suture base is 0.5 mm to 1.5 mm.

5.  The polymeric mitral heart valve according to claim 3, wherein the insert has a frame structure matching the outer contour of the stent frame, and protruding fixing ribs and planar fixing ribs are circumferentially arranged on the insert corresponding to the upper edges of the protruding sections and the planar section, respectively; first suture holes are arranged on both the protruding fixing ribs and the planar fixing ribs, and the first suture holes and the through holes are circumferentially arranged in a one-to-one correspondence.

6.  The polymeric mitral heart valve according to claim 1, wherein the insert comprises a plurality of positioning peaks, a plurality of positioning teeth, and a plurality of positioning protrusions; the positioning peaks are arranged at an end adjacent to the first end, and the plurality of positioning peaks are circumferentially arranged corresponding to the strut peaks; the positioning teeth are arranged at an end adjacent to the second end, and the circumferential positions of the plurality of positioning teeth correspond one-to-one to the strut peaks and the strut valleys; the positioning protrusions are uniformly arranged on the outer wall of the insert.

7.  The polymeric mitral heart valve according to claim 6, wherein second suture holes are arranged at the tops of the positioning peaks.

8.  The polymeric mitral heart valve according to claim 1, wherein each leaflet comprises a free edge and an attachment edge connected to the stent frame; in a natural state, gaps are reserved between the free edges of adjacent leaflets, a concave structure is formed between the midpoint of each free edge and the endpoints of the attachment edge, and the leaflets are semi-open.

9.  The polymeric mitral heart valve according to claim 8, wherein the line connecting the midpoint of the free edge and the midpoint of the attachment edge forms a belly straight line on each leaflet, and the angle between the belly straight line and the cross-section of the stent frame is 30° to 45°.

10. The polymeric mitral heart valve according to claim 8, wherein M is defined as the midpoint of the connecting arc extending circumferentially along the stent frame between two endpoints of the attachment edge, and N is defined as the commissural point of the leaflets in a natural state; the line connecting M and N extends obliquely from the first end to the second end, and the angle between the line connecting M and N and the cross-section of the stent frame is 10° to 30°.

11. The polymeric mitral heart valve according to claim 8, wherein the free edge of a single leaflet is located in a coordinate plane, where the X-axis of the coordinate system is defined by the line connecting the midpoint of the free edge and one endpoint of the attachment edge, and the Y-axis is perpendicular to the connecting line and passes through the midpoint of the free edge; the parametric curve Y of the free edge from the midpoint to one endpoint of the attachment edge is represented by the following formula:

$$Y = \begin{cases} H_1 \sin\left(\dfrac{X}{L_1}\pi\right) & 0 \leq X \leq L_1 \\[2ex] H_2 \sin\left(\dfrac{X}{L_1 + L_2}3\pi\right) & L_1 < X < L_2 \\[2ex] H_3 \sin\left(\dfrac{X}{L_2 + L_3}\pi\right) & L_2 \leq X \leq L_3 \end{cases}$$

wherein $L_1$ and $L_2$ are adjustable parameters and satisfy $L_3/10 \leq L_1 \leq L_3/5$ and $2L_3/3 \leq L_2 \leq 3L_3/4$, respectively; $L_3$ is a linear distance between the midpoint of the free edge and one endpoint of the attachment edge; $H_1$, $H_2$, and $H_3$ are adjustable parameters and satisfy $0.05$ mm $\leq H_1 \leq 0.20$ mm, $0.20$ mm $\leq H_2 \leq 1.0$ mm, and $0$ mm $\leq H_3 \leq 0.25$ mm, respectively.

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

FIG. 11

## INTERNATIONAL SEARCH REPORT

| International application No. |
|---|
| **PCT/CN2024/126038** |

### A. CLASSIFICATION OF SUBJECT MATTER

A61F2/24(2006.01)i

According to International Patent Classification (IPC) or to both national classification and IPC

### B. FIELDS SEARCHED

Minimum documentation searched (classification system followed by classification symbols)

IPC: A61F2

Documentation searched other than minimum documentation to the extent that such documents are included in the fields searched

Electronic data base consulted during the international search (name of data base and, where practicable, search terms used)

CNTXT, ENTXTC, VEN: 瓣膜, 瓣环, 缝合环, 缝合带, 凸起, 突起, 凸出部, 突出部, 瓣叶, 小叶, 瓣架, 一次, 一体, 成型, 嵌件, 半开口, 半闭合, 不完全, 闭合, 关闭, heart, valve, leaflet?, suture, sewing, frame, ring, protrud+, project+, single, one, time, inject+, mold+, insert, half, semi, incomplete, open, close

### C. DOCUMENTS CONSIDERED TO BE RELEVANT

| Category* | Citation of document, with indication, where appropriate, of the relevant passages | Relevant to claim No. |
|---|---|---|
| PX | CN 117257524 A (SUZHOU XINLING MAIDE MEDICAL TECHNOLOGY CO., LTD.) 22 December 2023 (2023-12-22) <br> claims 1-11, description, paragraphs 30-50, and figures 1-11 | 1-11 |
| Y | CN 116509602 A (SUZHOU XINLING MAIDE MEDICAL TECHNOLOGY CO., LTD.) 01 August 2023 (2023-08-01) <br> description, paragraphs 36-47, and figures 1-4 | 1-10 |
| Y | CN 101217920 A (EDWARDS LIFESCIENCES CORP.) 09 July 2008 (2008-07-09) <br> description, paragraphs 39-67, and figures 1-12 | 1-10 |
| Y | CN 115105261 A (SHANGHAI YIXIN MEDICAL DEVICES CO., LTD.) 27 September 2022 (2022-09-27) <br> description, paragraphs 49-51, and figures 1-11 | 8-10 |
| Y | CN 115778635 A (SHANGHAI LANFAN BOAO MEDICAL TECHNOLOGY CO., LTD.) 14 March 2023 (2023-03-14) <br> description, paragraphs 30-36, and figures 1-2 | 8-9 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **14 January 2025** | **13 February 2025** |

| Name and mailing address of the ISA/CN | Authorized officer |
|---|---|
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/126038** |

| C. | DOCUMENTS CONSIDERED TO BE RELEVANT | |
| --- | --- | --- |
| Category* | Citation of document, with indication, where appropriate, of the relevant passages | Relevant to claim No. |
| A | US 6283995 B1 (SULZER CARBOMEDICS INC.) 04 September 2001 (2001-09-04)<br>entire document | 1-11 |

Form PCT/ISA/210 (second sheet) (July 2022)

## INTERNATIONAL SEARCH REPORT
### Information on patent family members

International application No.

**PCT/CN2024/126038**

| Patent document cited in search report | | | Publication date (day/month/year) | Patent family member(s) | | | Publication date (day/month/year) |
|---|---|---|---|---|---|---|---|
| CN | 117257524 | A | 22 December 2023 | CN | 117257524 | B | 15 March 2024 |
| CN | 116509602 | A | 01 August 2023 | CN | 116509602 | B | 06 February 2024 |
| | | | | WO | 2024245122 | A1 | 05 December 2024 |
| CN | 101217920 | A | 09 July 2008 | EP | 1977718 | A2 | 08 October 2008 |
| | | | | EP | 1977718 | A3 | 27 April 2011 |
| | | | | EP | 1977718 | B1 | 30 July 2014 |
| | | | | EP | 1901682 | A2 | 26 March 2008 |
| | | | | EP | 1901682 | B1 | 08 May 2013 |
| | | | | CA | 2610727 | A1 | 18 January 2007 |
| | | | | CA | 2610727 | C | 05 November 2013 |
| | | | | WO | 2007008371 | A2 | 18 January 2007 |
| | | | | WO | 2007008371 | A3 | 22 March 2007 |
| | | | | JP | 2009501058 | A | 15 January 2009 |
| | | | | JP | 5285425 | B2 | 11 September 2013 |
| | | | | US | 2007016289 | A1 | 18 January 2007 |
| | | | | US | 7776084 | B2 | 17 August 2010 |
| | | | | CN | 101217920 | B | 16 March 2011 |
| | | | | CN | 102028565 | A | 27 April 2011 |
| | | | | CN | 102028565 | B | 18 July 2012 |
| | | | | JP | 2013039428 | A | 28 February 2013 |
| CN | 115105261 | A | 27 September 2022 | CN | 218528988 | U | 28 February 2023 |
| CN | 115778635 | A | 14 March 2023 | CN | 115778635 | B | 26 May 2023 |
| | | | | WO | 2024160194 | A1 | 08 August 2024 |
| US | 6283995 | B1 | 04 September 2001 | WO | 0062714 | A1 | 26 October 2000 |
| | | | | JP | 2002541913 | A | 10 December 2002 |
| | | | | EP | 1171061 | A1 | 16 January 2002 |

Form PCT/ISA/210 (patent family annex) (July 2022)

**EP 4 781 949 A1**

**REFERENCES CITED IN THE DESCRIPTION**

**Patent documents cited in the description**

- CN 202311394285